# EUROPEAN PATENT APPLICATION

(11) **EP 2 140 814 A1**
(43) Date of publication of application: **06.01.2010**
(21) Application number: 08159605.8
(22) Date of filing: 03.07.2008
(51) Int. Cl.: A61B 10/02, A61B 17/322, G01N 1/04, G01N 1/06, G01N 1/42, A61B 17/32

(54) **Appliance for collecting and cryopreserving a tissue piece**

(71) Applicant: Kabushiki Kaisha Kitazato Supply, Fujinomiya-shi, Shizuoka 418-0039 (JP)
(72) Inventor: Kagawa, Noriko, Tokyo Tokyo 160-0023 (JP); Kuwayama, Masashige, Tokyo 160-0023 (JP); Inoue, Futoshi, Fujinomiya-shi Shizuoka 418-0039 (JP)
(74) Representative: Dossmann, Gérard

(57) **Abstract**

An appliance (1) for collecting and cryopreserving a tissue piece has a cutting position-specifying tool (10) and a tissue piece cryopreserving plate (4) for cryopreserving a tissue piece cut from an organ tissue by the cutting position-specifying tool (10). The cutting position-specifying tool (10) has a tissue cutting portion-specifying member (2) having a cutting portion-specifying open portion (22) flat plate-shaped and having a predetermined width and a locking portion (23) for locking an advance of a cutter provided on a lower surface of the tissue cutting portion-specifying member (2 and an open portion-pressing member (3) which can be pressed against an upper surface of the tissue cutting portion-specifying member (2) in such a way that the open portion-pressing member (3) covers the cuffing portion-specifying open portion (22). The tissue piece cryopreserving plate (4) is a plate-shaped metal member which has a width equal to or a little larger than the predetermined width of the cuffing portion-specifying open portion (22), is extended in a predetermined length, allows the tissue piece cut from the organ tissue by the cutting position-specifying tool (10) to be placed thereon, and has a lot of holes allowing a cooling medium to penetrate thereinto.

## Description

The present invention relates to an appliance for cryopreserving a collected tissue piece by vitrification. The appliance is used in cryopreserving tissues of organisms such as the tissue of the ovary of a mammal.

Currently cells (for example, spermatozoon, ovum, and the like) of the body of the mammal are cryopreserved. The freeze preservation of the body cells enables the preservation of hereditary resources of a specified system and kind and is effective for maintaining animals on the verge of extinction and for sterile treatment

A method of freezing the ovary is disclosed in Japanese Patent Application Laid-Open No. 2003-284546. In this method, a reproductive tissue including at least the ovary taken out from a female individual of an impersonal mammal is preserved at 15 to 0°C and thereafter frozen by a vitrification method.

In the method of cryopreserving an embryo of the mammal disclosed in Japanese Patent Application Laid-Open No. 2000-189155, the embryo or an ovum is stuck to the inner surface of a sterilized cryopreserving container such as a freezing straw, a freezing vial, a freezing tube, and the like with the embryo or the ovum immersed in a minimum amount of a vitrification solution sufficient for covering the embryos or the ova, the cryopreserving container is sealed, and the cryopreserving container is brought into contact with liquid nitrogen to rapidly cool the cryopreserving container. In the melting method disclosed in Japanese Patent Application Laid-Open No. 2000-189155, the cryopreserving container preserved by the above-described method is taken out of the liquid nitrogen, one end of the container is opened, a dilution solution having a temperature of 33 to 39°C is directly injected into the container to melt and dilute the frozen embryo or ovum. This method has an excellent effect because there is no fear that the embryo or the ovum of the mammal is infected by virus or bacteria and can be preserved and melted and diluted at a high survival percentage.

The present applicant proposed an appliance for cryopreserving the embryo, as disclosed in WO02085110. The appliance for cryopreserving the embryo includes a main body made of a material resistant to cold; a strip, made of the material flexible, transparent, and resistant to liquid nitrogen, where the embryo sticks and is held; and a cylindrical member, made of the material resistant to cold, which is sealed at one end thereof and removably mounted on the main body: The cylindrical member is capable of covering the strip.

Only the method of freezing the ovary is disclosed in Japanese Patent Application Laid-Open No. 2003-284546. No disclosure is made on a simple appliance for collecting and cryopreserving tissues. The methods disclosed in Japanese Patent Application Laid-Open Nos. 2000-189155 and 2002-315573 cannot be utilized to collect and cryopreserve tissues.

The present invention has been made to solve the above-described problems. Therefore it is an object of the present invention to provide an appliance, a plate, and a method capable of easily collecting a tissue piece and preferably cryopreserving the collected tissue piece by vitrification.

The above-described object is achieved by an appliance for cryopreserving a collected tissue piece.

An appliance for cryopreserving a collected tissue piece comprises a cutting position-specifying tool and a tissue piece cryopreserving plate for cryopreserving a tissue piece cut from an organ tissue by using said cutting position-specifying tool, wherein said cutting position-specifying tool includes a tissue cutting portion-specifying member having a cutting portion-specifying open portion which is flat plate-shaped and has a predetermined width and a locking portion, for locking an advance of a cutter, which is provided on a lower surface of said tissue cutting portion-specifying member; and an open portion-pressing member which can be pressed against an upper surface of said tissue cutting portion-specifying member in such a way that said open portion-pressing member covers said cutting portion-specifying open portion; and said tissue piece cryopreserving plate is a plate-shaped metal member which has a width equal to or a little larger than said predetermined width of said cutting portion-specifying open portion, is extended in a predetermined length, allows said tissue piece cut from said organ tissue by using said collected tissue cutting position-specifying tool to be placed thereon, and has holes allowing a cooling medium to penetrate thereinto.
Fig. 1 is a front view of one embodiment of an appliance of the present invention for cryopreserving a collected tissue piece by a vitrification method.
Fig. 2 is a rear view of a tissue cutting portion-specifying member of the appliance shown in Fig. 1.
Fig. 3 is a sectional view taken along a line A-A of Fig. 1.
Fig. 4 is a sectional view taken along a line B-B of Fig. 1.
Fig. 5 is a sectional view taken along a line C-C of Fig. 1.
Fig. 6 is a rear view of a tissue cutting portion-specifying member for use in an appliance of another embodiment of the present invention.
Fig. 7 is a rear view of a tissue cutting portion-specifying member for use in an appliance of still another embodiment of the present invention.
Fig. 8 is a rear view of a tissue cutting portion-specifying member for use in an appliance of still another embodiment of the present invention.
Fig. 9 is a front view of a tissue cutting portion-specifying member for use in an appliance of still another embodiment of the present invention.
Fig. 10 is a sectional view taken along a line D-D of Fig. 9.
Fig. 11 is an explanatory view for explaining the operation of the appliance of the embodiment shown in Fig. 9.
Fig. 12 is front view of a tissue cutting portion-specifying member for use in an appliance of still another embodiment of the present invention.
Fig. 13 is a front view of an open portion-pressing member for use in the appliance of the embodiment shown in Fig. 12.
Fig. 14 is a sectional view taken along a line E-E of Fig. 12.
Fig. 15 is a sectional view taken along a line F-F of Fig. 12.
Fig. 16 is an explanatory view for explaining the operation of the appliance of the embodiment shown in Figs. 12 through 14.
Fig. 17 is an explanatory view for explaining the operation of the appliance of the embodiment shown in Figs. 12 through 14.
Fig. 18 is a rear view of a tissue cutting portion-specifying member for use in an appliance of still another embodiment of the present invention.
Fig. 19 is an enlarged plan view of the appliance shown in Fig. 18.
Fig. 20 is a sectional view taken along a line G-G of Fig. 18.
Fig. 21 is an explanatory view for explaining the operation of the appliance of the embodiment shown in Figs. 18 and 19.
Fig. 22 is a front view of a tissue piece cryopreserving plate for use in an appliance of still another embodiment of the present invention.
Fig. 23 is a front view of a tissue piece cryopreserving plate for use in an appliance of still another embodiment of the present invention.

The embodiments of the appliance and method of the present invention for cryopreserving a collected tissue piece by vitrification (in other word, vitrification cryopreserving a collected tissue piece) are described below with reference to the drawings.

An appliance 1 of the present invention for cryopreserving a collected tissue piece has a cutting position-specifying tool (collected tissue cutting position-specifying tool) 10 and a tissue piece cryopreserving plate 4 for cryopreserving a tissue piece cut from tissues of an organ by the collected tissue cutting position-specifying tool 10.

The collected tissue cutting position-specifying tool 10 includes a tissue cutting portion-specifying member 2 having a cutting portion-specifying open portion 22 flat plate-shaped and having a predetermined width and a locking portion 23, for locking an advance of a cutter, which is provided on a lower surface of the tissue cutting portion-specifying member 2; and an open portion-pressing member 3 which can be pressed against an upper surface of the tissue cutting portion-specifying member 2 in such a way that the open portion-pressing member 3 covers the cutting portion-specifying open portion 22. The tissue piece cryopreserving plate 4 is a plate-shaped metal member which has a width equal to or a little larger than the predetermined width of the cutting portion-specifying open portion 22, is extended in a predetermined length, allows the tissue piece cut from the organ tissues by the collected tissue cutting position-specifying tool 10 to be placed thereon, and has a lot of (a large number of) holes allowing a cooling medium to penetrate thereinto.

The tissue piece cryopreserving plate 4 of the present invention is a plate-shaped metal member which is extended in the predetermined length and has a tissue piece-placing portion having a lot of holes formed therein to allow the cooling medium to penetrate thereinto.

The appliance of the present invention for cryopreserving a collected tissue piece by vitrification and the tissue piece cryopreserving plate of the present invention can be used to cryopreserve collected various tissues of various organisms such as cows, human being, and the like by vitrification and are especially preferable for cryopreserving tissue pieces of the ovary by vitrification.

In the appliance 1 of the present invention for cryopreserving a collected tissue piece by vitrification, after an organ tissue is notched along an inner edge of the cutting portion-specifying open portion 22 with the tissue cutting portion-specifying member 2 being pressed against the organ tissue, the open portion-pressing member 3 is pressed against an upper surface of the tissue cutting portion-specifying member 2 in such a way that the open portion-pressing member 3 covers the cutting portion-specifying open portion 22. With the open portion-pressing member 3 being pressed against a portion of the organ tissue surrounded with a notched line, a cutter is moved along a lower surface of the tissue cutting portion-specifying member 2. Thereby it is possible to cut a tissue piece having a configuration corresponding to that of the open portion 22 from the organ tissue.

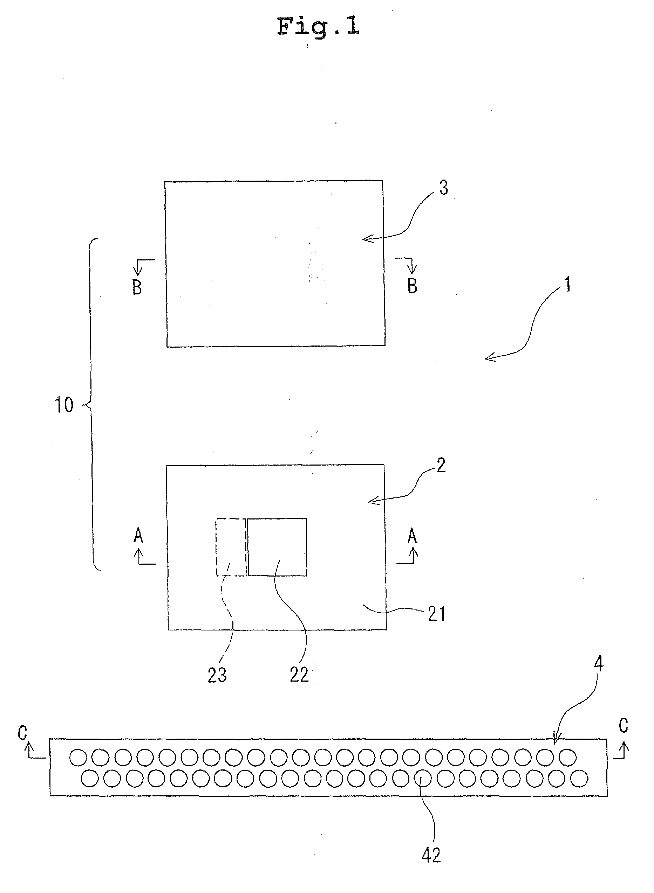

As shown in Fig. 1, the appliance 1 of the present invention for cryopreserving a collected tissue piece by vitrification has the collected tissue cutting position-specifying tool 10 and the tissue piece cryopreserving plate 4 for cryopreserving a tissue piece cut from an organ tissue by the collected tissue cutting position-specifying tool 10. In this embodiment, the appliance 1 is composed of the set of the collected tissue cutting position-specifying tool 10 and the tissue piece cryopreserving plate 4. But the tissue piece cryopreserving plate 4 of the present invention can be used for a tissue piece collected without using the collected tissue cutting position-specifying tool 10.

The collected tissue cutting position-specifying tool 10 has the tissue cutting portion-specifying member 2 and the open portion-pressing member 3.

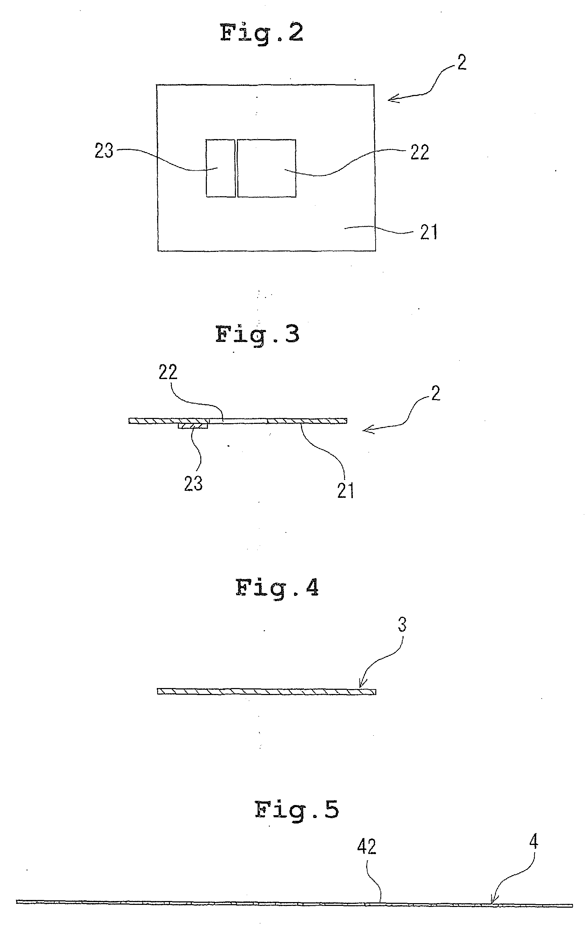

As shown in Figs. 1 through 3, the tissue cutting portion-specifying member 2 is a flat plate-shaped member formed approximately rectangularly. The upper and lower surfaces of the tissue cutting portion-specifying member 2 are flat. The rectangular cutting portion-specifying open portion 22 that extends in a predetermined width and length is formed at a central portion of a flat part 21 of the tissue cutting portion-specifying member 2.

In this embodiment, the cutting portion-specifying open portion 22 is rectangular. The tissue piece cryopreserving plate 4 which is described later has a width equal to or a little larger than that of the rectangular open portion 22 and has a length much longer than that of the rectangular open portion 22.

The tissue cutting portion-specifying member 2 has the locking portion 23, for locking the advance of a cutter, which is formed on the lower surface thereof in the neighborhood of the cutting portion-specifying open portion 22, with the locking portion 23 disposed at one or other end thereof with respect to the open portion 22. The locking portion 23 for locking the advance of the cutter is projected from the flat lower surface of the tissue cutting portion-specifying member 2. In this embodiment, the locking portion 23 is disposed between the other end of the tissue cutting portion-specifying member 2 and the cutting portion-specifying open portion 22 with the locking portion 23 located proximately to the cutting portion-specifying open portion 22. It is preferable to dispose the locking portion 23 in the neighborhood of the cutting portion-specifying open portion 22.

As the size of the tissue cutting portion-specifying member 2, supposing that the tissue cutting portion-specifying member is rectangular, it is preferable that the longer side thereof is 30 to 50mm and the shorter side thereof is 20 to 40mm. The thickness of the tissue cutting portion-specifying member 2 is preferably 0.5 to 2mm. The thickness of the locking portion 23 for locking the advance of a cutter is preferably 0.5 to 2mm. The size of the cutting portion-specifying open portion 22 is preferably 25 to 250mm². Supposing that the cutting portion-specifying open portion is rectangular, the lengths of the sides are preferably 5 to 15mm.

It is favorable that materials for forming the tissue cutting portion-specifying member 2 are transparent or semitransparent and are not adhesive to an organ tissue. Thus as the materials for forming the tissue cutting portion-specifying member 2, the following substances can be preferably used: polyolefin (for example, polyethylene, polypropylene, ethylene-propylene copolymer, and ethylene-vinyl acetate copolymer); styrene resin (for example, polystyrene, methacrylate-styrene copolymer, methacrylate-butylene-styrene copolymer); polyester (for example, polyethylene terephthalate, polybutylene terephthalate); polyamide (for example, nylon 6, nylon 66); and fluororesins such as polytetrafluoroethylene, ethylene-tetrafluoroethylene copolymer, tetrafluoroethylene-hexafluoropropylene copolymer, chlorotrifluoroethylene resin.

A transparent flat plate-shaped member is used for the open portion-pressing member 3. In this embodiment, the open portion-pressing member 3 is rectangular in correspondence to the configuration of the tissue cutting portion-specifying member 2. The open portion-pressing member 3 can be pressed against the upper surface of the tissue cutting portion-specifying member 2 in such a way that the open portion-pressing member 3 covers the cutting portion-specifying open portion 22. Like the open portion-pressing member 3 of this embodiment, it is preferable that the open portion-pressing member 3 has a configuration corresponding to the outer configuration of the tissue cutting portion-specifying member 2. The open portion-pressing member 3 may be a little smaller or larger than the tissue cutting portion-specifying member 2.

As the size of the open portion-pressing member 3, supposing that the open portion-pressing member 3 is rectangular, it is preferable that the longer side thereof is 30 to 50mm and that the shorter side thereof is 20 to 40mm. The thickness of the open portion-pressing member 3 is preferably 0.5 to 2mm. As materials for forming the open portion-pressing member 3, it is preferable to use the above-described materials for the tissue cutting portion-specifying member 2.

The tissue piece cryopreserving plate 4 extended in a predetermined length has the shape of a flat plate having a width equal to or a little larger than the predetermined width of the cutting portion-specifying open portion 22 and allows the tissue piece cut from the organ tissue by using the collected tissue cutting position-specifying tool 10 to be placed thereon.

In this embodiment, the tissue piece cryopreserving plate 4 has the shape of a long and narrow flat plate having a width substantially equal to the length of a side of the cutting portion-specifying open portion 22. When the tissue piece cryopreserving plate 4 is rectangular, it is preferable that the longer side thereof is set to 50 to 150mm and that the shorter side thereof is set to 5 to 50mm.

The tissue piece cryopreserving plate 4 has a lot of holes 42 allowing the cooling medium to be inserted thereinto. By providing the tissue piece cryopreserving plate 4 with a lot of the holes 42 allowing the cooling medium to be inserted thereinto, the plate 4 rapidly cools and in addition the cooling medium is capable of contacting a portion of the tissue piece placed at the side of the plate 4. Thus the tissue piece can be preferably cooled from the portion thereof disposed at the side of the plate 4. Owing to these two effects, the tissue piece can be cooled at a very high speed. By using the tissue cutting portion-specifying member 2 and th open portion-pressing member 3, it is possible to collect the tissue piece corresponding to the configuration of the tissue piece cryopreserving plate 4 and having a desired precise thickness. Owing to the complex operation and effect, it is possible to obtain cells at a high survival percentage when the cells are thawed after they are cryopreserved by vitrification. Therefore the tissue piece cryopreserving plate 4 is very effective for autotransplantation.

As shown in Fig. 1, in this embodiment, the tissue piece cryopreserving plate 4 is an approximately rectangular thin plate and entirely has a lot of holes allowing the cooling medium to be inserted thereinto. It is preferable that the holes 42 of the tissue piece cryopreserving plate 4 are circular. The size of each of the holes is favorably 2 to 8mm² and more favorably 4 to 7mm². The ratio of the total area of the holes to the entire area of the tissue piece cryopreserving plate 4 is set to favorably 30 to 70%. The tissue piece cryopreserving plate 4 is made of a flat plate-shaped metal. As metals for the tissue piece cryopreserving plate 4, copper, copper alloy, aluminum, aluminum alloy, gold, gold alloy, silver, and silver alloy are preferable, although not specifically limited. It is preferable that the cooling speed of the tissue piece cryopreserving plate 4 is not less than -20,000°C in terms of one minute, when the tissue piece cryopreserving plate 4 is immersed in liquid nitrogen. It is possible to freeze the tissue piece in a vitrified state when the tissue piece cryopreserving plate 4 is cooled at the above-described cooling speed.

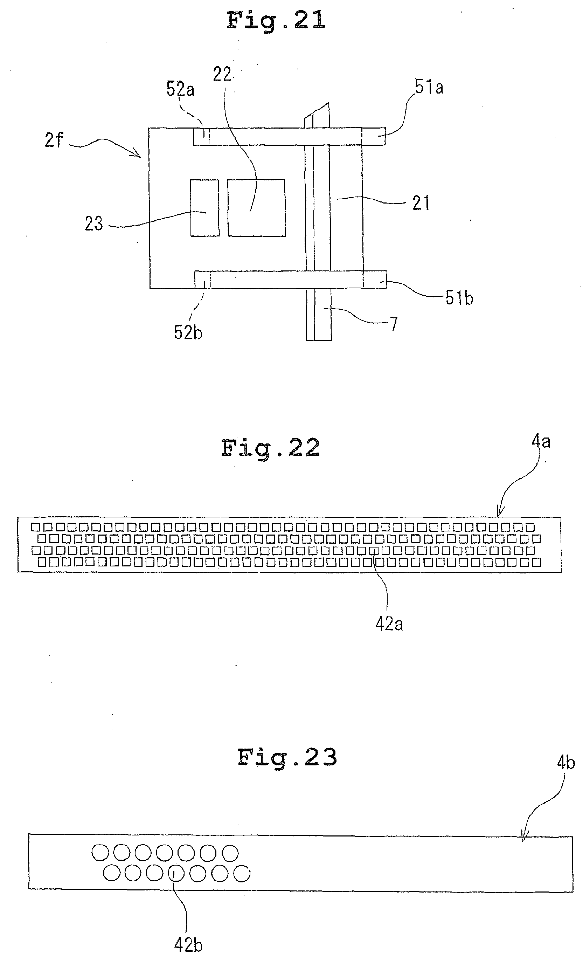

The configuration of the hole to be formed in the tissue piece cryopreserving plate 4 is not limited to the circular type as shown in Fig. 1, but a polygonal (in this embodiment, quadrangular) hole 42a may be formed in a tissue piece cryopreserving plate 4a as shown in Fig. 22.

As shown in Fig. 23, holes may be formed in the tissue piece cryopreserving plate in only a region where the tissue piece is placed. In a tissue piece cryopreserving plate 4b of the embodiment shown in Figs. 23, holes 42b are formed in only a predetermined region disposed at one side thereof. It is preferable that the longitudinal length of the predetermined region of the plate 4b in which the holes 42b are formed are set to 2 to 5 times larger than the length of the cutting portion-specifying open portion 22. It is preferable that the size of the hole 42b is equal to that described above. The configuration of the hole 42b may be polygonal, as shown in Fig. 22.

Another embodiment of the present invention is described below.

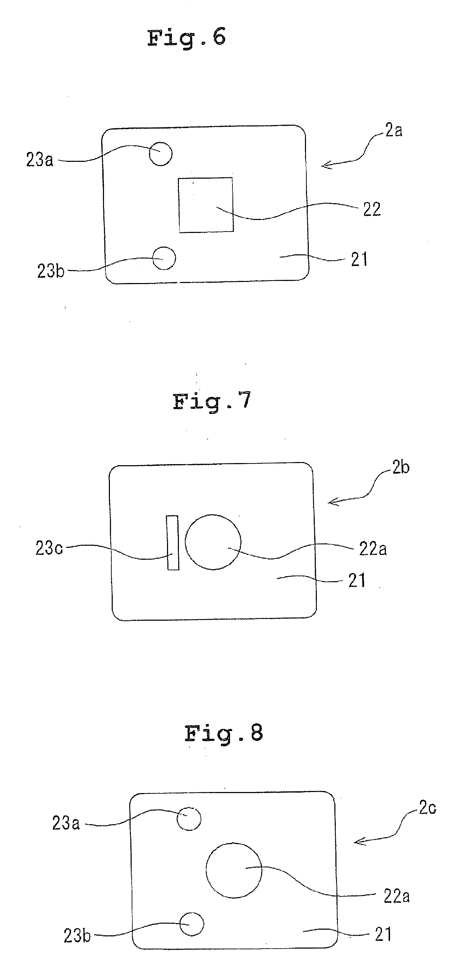

Figs. 6 through 8 are rear views of tissue cutting portion-specifying members for use in appliances of other embodiments of the present invention for cryopreserving a collected tissue piece by vitrification.

Corners of a tissue cutting portion-specifying member 2a may be chamfered, as shown in Fig. 6. The form of the locking portion 23 for locking the advance of the cutter is not limited to that shown in Figs. 1 through 3, but like locking portions 23a, 23b shown in Fig. 6, locking portions 23a, 23b may be disposed between the other end of the tissue cutting portion-specifying member 2a and the cutting portion-specifying open portion 22 with the locking portions 23a, 23b disposed at both sides of the tissue cutting portion-specifying member 2a. As shown in Fig. 6, the locking portion 23a, 23b may be circular, elliptic, polygonal or the like.

The configuration of the cutting portion-specifying open portion of the collected tissue cutting position-specifying tool is not limited to that shown in Figs. 1 through 3, but the cutting portion-specifying open portion may be circular or elliptic like an open portion 22a of a tissue cutting portion-specifying member 2b of the embodiment shown in Fig. 7. As shown in Fig. 7, a locking portion 23c for locking the advance of the cutter is disposed between the other end of the tissue cutting portion-specifying member 2b and the cutting portion-specifying open portion 22a.

In using the tissue cutting portion-specifying member 2b of such a type, the tissue piece cryopreserving plate that can be used has a width equal to or a little larger than the diameter or the minor axis of the open portion 22a and has a length much longer than the diameter or the major axis of the open portion 22a.

As shown by a tissue cutting portion-specifying member 2c of the embodiment shown in Fig. 8, it is possible that a cutting portion-specifying open portion 22a is circular or elliptic and that locking portions 23a, 23b may be disposed between the other end of a tissue cutting portion-specifying member 2c and the cutting portion-specifying open portion 22a with the locking portions 23a, 23b disposed at both sides of the tissue cutting portion-specifying member 2c. The locking portions 23a, 23b may be circular, elliptic, polygonal or the like.

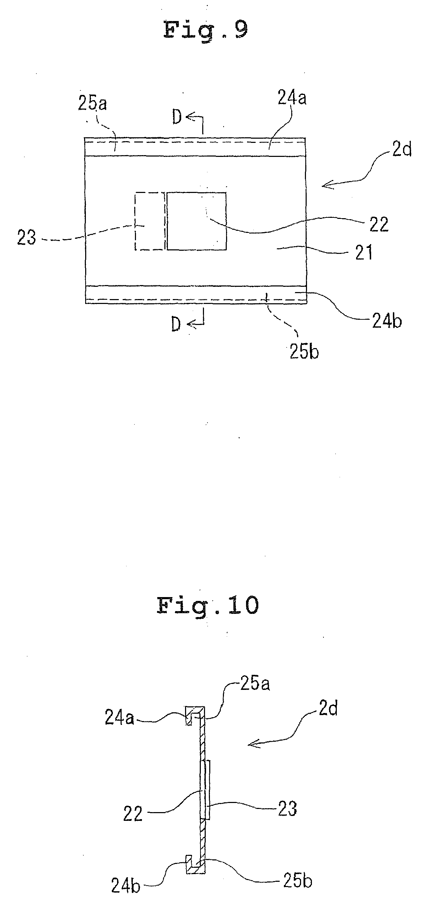

The above-described collected tissue cutting position-specifying tools may be provided with an open portion-pressing member guide portion 24a, 24b, as shown in Figs. 9 through 11.

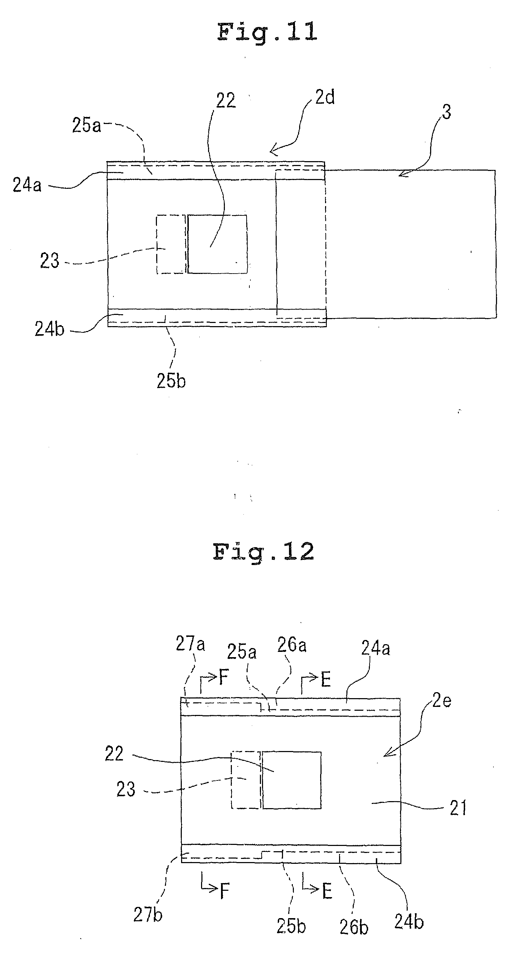

Fig. 9 is a front view of a tissue cutting portion-specifying member for use in an appliance of still another embodiment of the present invention for cryopreserving a collected tissue piece by vitrification. Fig. 10 is a sectional view taken along a line D-D of Fig. 9. Fig. 11 is an explanatory view for explaining the operation of the appliance of the embodiment shown in Fig. 9.

A tissue cutting portion-specifying member 2d of the embodiment shown in Figs. 9 through 11 has the open portion-pressing member guide portion 24a, 24b which is disposed at both sides of the upper surface thereof in the range from one longitudinal end thereof to the other end thereof and slidably accommodates both sides of the open portion-pressing member 3. The open portion-pressing member guide portion 24a, 24b extends from one longitudinal end of the tissue cutting portion-specifying member 2d to the other longitudinal end thereof. In this embodiment, the open portion-pressing member guide portions 24a, 24b extend from the one longitudinal end of the tissue cutting portion-specifying member 2d to the other longitudinal end thereof over the full length thereof. As shown in Fig. 10, the open portion-pressing member guide portions 24a, 24b have a concave portion 25a, 25b respectively accommodating both sides of the open portion-pressing member 3. Both sides of the open portion-pressing member 3 are slidably accommodated in the concave portion 25a, 25b. The open portion-pressing member guide portion 24a, 24b covers an upper portion of the concave portion 25a, 25b, thus preventing the open portion-pressing member 3 from separating from the tissue cutting portion-specifying member 2d.

As shown in Fig. 11, in the collected tissue cutting position-specifying tool of this embodiment, let it be supposed that one end of the open portion-pressing member 3 has advanced to a certain position of the tissue cutting portion-specifying member 2d where the open portion-pressing member 3 does not cover the cutting portion-specifying open portion 22, with the widthwise ends of the open portion-pressing member 3 penetrating in the open portion-pressing member guide portion 24a, 24b of the tissue cutting portion-specifying member 2d respectively. In this state, with the collected tissue cutting position-specifying tool pressed against a out organ (for example, ovary, liver) taken out, the organ is notched by using a cutter along the inner edge of the cutting portion-specifying open portion 22. Thereby a portion of the organ surrounded with a notched line penetrates into the open portion 22. Thereafter the open portion-pressing member 3 is slid to cover the cutting portion-specifying open portion 22 with the open portion-pressing member 3. In this embodiment, the provision of the open portion-pressing member guide portion 24a, 24b facilitates a work of disposing the open portion-pressing member 3 on the cutting portion-specifying open portion 22.

With the open portion-pressing member 3 being pressed against the tissue cutting portion-specifying member 2d, the cutter is moved toward the locking portion 23 for locking the advance of the cutter along the lower surface of the tissue cutting portion-specifying member 2d. Thereby a tissue piece having a configuration corresponding to that of the cutting portion-specifying open portion 22 is cut from the organ.

All of the above-described collected tissue cutting position-specifying tools may have a construction as shown in Figs. 12 through 17.

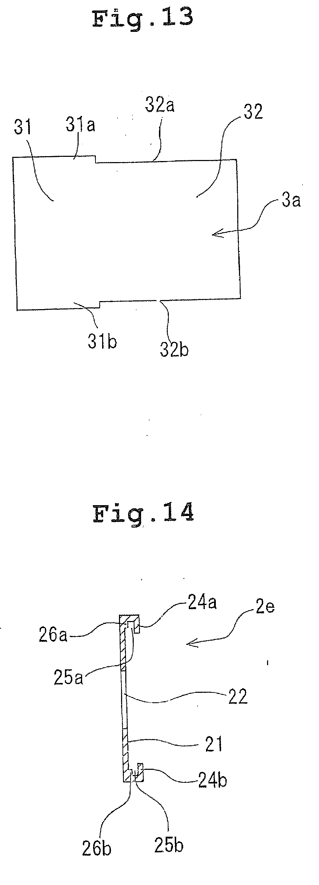

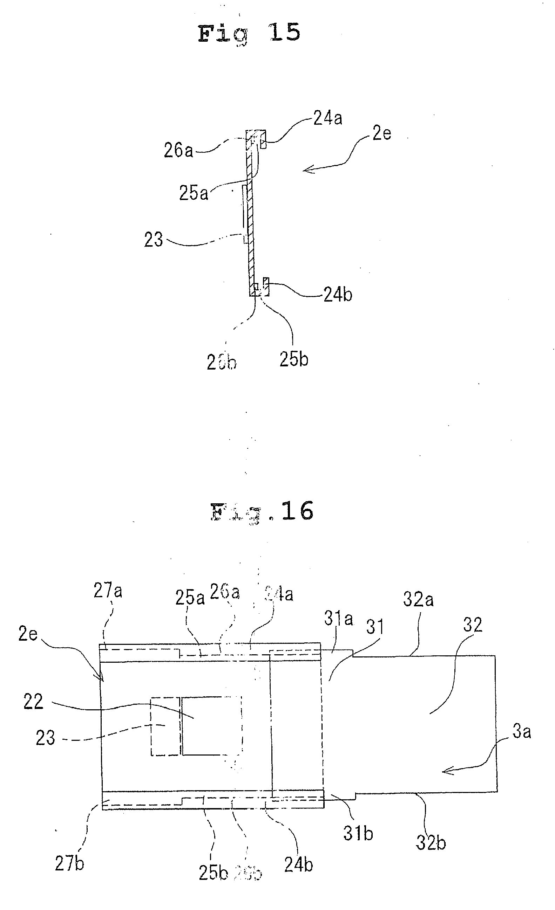

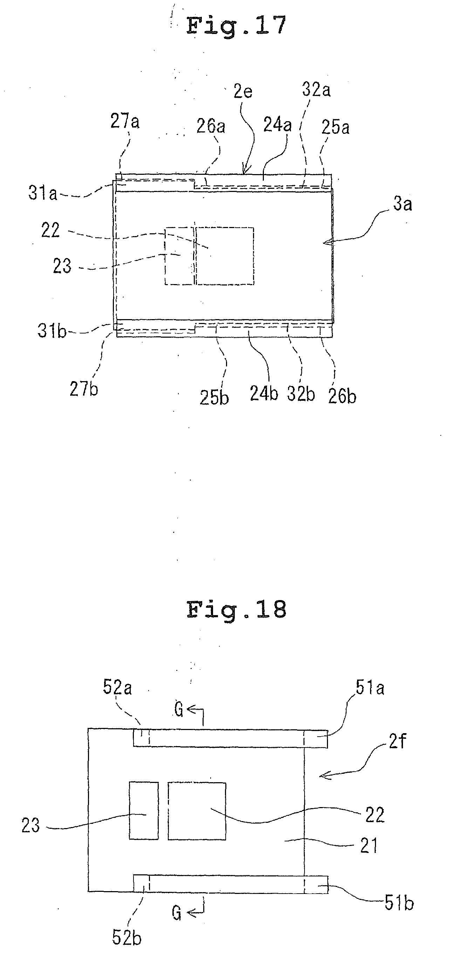

Fig. 12 is a front view of a tissue cutting portion-specifying member for use in an appliance of still another embodiment of the present invention for cryopreserving a collected tissue piece by vitrification. Fig. 13 is a front view of an open portion-pressing member for use in the appliance of the embodiment shown in Fig. 12. Fig. 14 is a sectional view taken along a line E-E of Fig. 12. Fig. 15 is a sectional view taken along a line F-F of Fig. 12. Figs. 16 and 17 are explanatory views for explaining the operation of the appliance of the embodiment shown in Figs. 12 through 15.

Similarly to the above-described tissue cutting portion-specifying member 2d, a tissue cutting portion-specifying member 2e of the embodiment has the open portion-pressing member guide portion 24a, 24b which is disposed at both sides of the upper surface thereof in the range from one longitudinal end thereof to the other end thereof and slidably accommodates both sides of the open portion-pressing member 3a. The open portion-pressing member guide portion 24a, 24b extends from one longitudinal end of the tissue cutting portion-specifying member 2d to the other longitudinal end thereof. In this embodiment, the open portion-pressing member guide portion 24a, 24b extends from the one longitudinal end of the tissue cutting portion-specifying member 2e to the other longitudinal end thereof over the full length thereof.

As shown in Figs. 12, 14, and 15, the open portion-pressing member guide portion 24a, 24b has the concave portion 25a, 25b accommodating both sides of the open portion-pressing member 3a and a stepped portion 26a, 26b extended from the upper surface of the tissue cutting portion-specifying member 2e to the concave portion 25a, 25b. A stepped portion-unformed portion 27a, 27b having a predetermined length is formed at one longitudinal end of the stepped portion 26a, 26b. As shown in Fig. 13, projected portions 31a, 31b are formed at both widthwise ends of the one longitudinal end of the open portion-pressing member 3a. Thereby the open portion-pressing member 3a has a wide portion 31 which has a predetermined longitudinal length and a larger width than the distance between the stepped portion 26a and stepped portion 26b of the tissue cutting portion-specifying member 2e and a smaller width than the distance between the stepped portion-unformed portion 27a and stepped portion-unformed portion 27b and a narrow portion 32 which is continuous with the wide portion 31 and has a cut-out portion 32a, 32b at both widthwise ends thereof. The width of the narrow portion 32 is shorter than the distance between the stepped portion 26a and stepped portion 26b of the tissue cutting portion-specifying member 2e. The widthwise ends of the wide portion 31 are accommodated inside the concave portion 25a, 25b with the widthwise ends thereof slidable inside the concave portion 25a, 25b and on the stepped portion 26a, 26b. The open portion-pressing member guide portions 24a, 24b cover the upper portion of the concave portion 25a, 25b, thus preventing the open portion-pressing member 3a from separating the tissue cutting portion-specifying member 2e.

As shown in Fig. 16, in the collected tissue cutting position-specifying tool of this embodiment, let it be supposed that one end of the open portion-pressing member 3a has advanced to a certain position of the tissue cutting portion-specifying member 2e where the open portion-pressing member 3a does not cover the cutting portion-specifying open portion 22, with the wide portion 31 of the open portion-pressing member 3a penetrating in the open portion-pressing member guide portion 24a, 24b of the tissue cutting portion-specifying member 2e. In this state, with the collected tissue cutting position-specifying tool pressed against an organ (for example, ovary, liver) taken out, the organ is notched by using the cutter along the inner edge of the cutting portion-specifying open portion 22. Thereby a portion of the organ surrounded with the notched line penetrates into the open portion 22. Thereafter the open portion-pressing member 3a is slid to cover the cutting portion-specifying open portion 22 with the open portion-pressing member 3a. In this embodiment, the open portion-pressing member 3a slides on the stepped portion 26a, 26b without substantially contacting the upper surface of the tissue cutting portion-specifying member 2e. Therefore the open portion-pressing member 3a slides without substantially contacting the portion of the organ which has penetrated into the open portion 22.

When the open portion-pressing member 3a has been advanced slidably and the entire wide portion 31 has reached the stepped portion-unformed portion 27a, 27b of the tissue cutting portion-specifying member 2e, as shown in Fig. 17, the wide portion 31 is capable of advancing into the stepped portion-unformed portions 27a, 27b. Thereby the open portion-pressing member 3a can be pressed against the tissue cutting portion-specifying member 2e. With the open portion-pressing member 3a being pressed against the tissue cutting portion-specifying member 2e, the cutter is moved toward the locking portion 23 for locking the advance of the cutter along the lower surface of the tissue cutting portion-specifying member 2e. Thereby a tissue piece having a configuration corresponding to that of the cutting portion-specifying open portion 22 is cut from the organ.

The above-described collected tissue cutting position-specifying tools may have a construction as shown in Figs. 18 through 21.

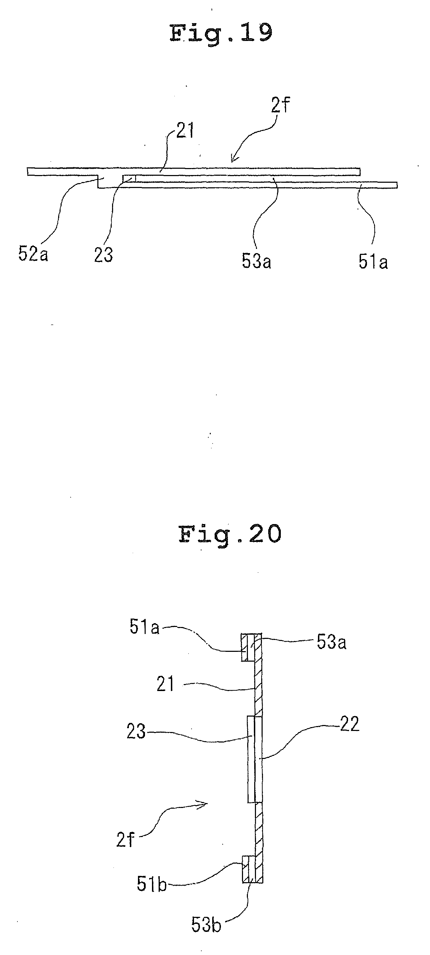

Fig. 18 is a rear view of a tissue cutting portion-specifying member for use in an appliance of still another embodiment of the present invention for cryopreserving a collected tissue piece by vitrification. Fig. 19 is an enlarged plan view of the appliance shown in Fig. 18. Fig. 20 is a sectional view taken along a line G-G of Fig. 18. Fig. 21 is an explanatory view for explaining the operation of the appliance of the embodiment shown in Figs. 18 and 19.

A tissue cutting portion-specifying member 2f of this embodiment has a cutter guide portion disposed at least one widthwise end of the lower surface thereof in the range from one longitudinal end thereof toward the other end thereof. The cutter is capable of sliding on the cutter guide portion. The provision of the cutter guide portion allows the tissue piece to be cut easily from a organ taken out. In this embodiment, the tissue cutting portion-specifying member 2f has a cutter guide portion 51a, 51b which are disposed at both widthwise ends of the lower surface thereof in the range from one longitudinal end thereof toward the other end thereof. The cutter is capable of sliding on the cutter guide portion. In this embodiment, the distal side of the cutter guide portion 51a, 51b is projected from the one longitudinal end of the tissue cutting portion-specifying member 2f to allow the cutter to be easily guided.

In the tissue cutting portion-specifying member 2f of this embodiment, the cutter guide portion 51a, 51b having a predetermined length is flat plate-shaped. As shown in Figs. 18 through 20, the distal side of the cutter guide portion 51a, 51b is formed as a free end, whereas the proximal end 52a, 52b thereof is fixed to a side portion of the tissue cutting portion-specifying member 2f corresponding to the portion where the locking portion 23 is formed. The cutter guide portion 51a, 51b does not substantially contact the lower surface of the tissue cutting portion-specifying member 2f. A gap 53a, 53b is formed between the lower surface of the tissue cutting portion-specifying member 2f and the cutter guide portion 51a, 51b. The distal side of the cutter guide portion 51a, 51b is projected from one end of the tissue cutting portion-specifying member 2f.

With the collected tissue cutting position-specifying tool pressed against an organ (for example, ovary, liver) taken out, the organ is notched by using the cutter along the inner edge of the cutting portion-specifying open portion 22. Thereby a portion of the organ surrounded with the notched line penetrates into the open portion 22. Thereafter with the open portion-pressing member 3 being pressed against the tissue cutting portion-specifying member 2f, a cutter 7 is placed on the cutter guide portion 51a, 51b projected from the one end of the tissue cutting portion-specifying member 2f. Thereafter the cutter 7 is moved toward the locking portion 23 for locking the advance of the cutter 7. As a result, as shown in Fig. 21 (rear view), the cutter 7 advances in the gap between the cutter guide portion 51a, 51b and the lower surface of the tissue cutting portion-specifying member 2f with the cutter 7 interposed therebetween. Thereby a tissue piece having a configuration corresponding to that of the cutting portion-specifying open portion 22 is cut from the organ.

The method of the present invention for cryopreserving a collected tissue piece by vitrification is described below.

The method of the present invention for cryopreserving the collected tissue by vitrification is carried out by using the appliance for cryopreserving a collected tissue piece by vitrification. It is possible to use any of the above-described appliances for cryopreserving the collected tissue piece by vitrification.

The method of the present invention for cryopreserving a collected tissue piece by vitrification includes the step of notching an organ tissue along an inner edge of a cutting portion-specifying open potion with a cutting portion-specifying member being pressed against the organ tissue, and pressing an open portion-pressing member against an upper surface of the cutting portion-specifying member, and with the open portion-pressing member being pressed against a portion of the organ tissue surrounded with a notched line, moving a cutter along a lower surface of the cutting portion-specifying member so as to cut a tissue piece having a configuration corresponding to that of the cutting portion-specifying open portion from the organ tissue, the step of immersing the collected tissue piece in a vitrification solution, the step of taking out the tissue piece from the vitrification solution and placing the tissue piece on a plate for cryopreserving the tissue piece, and the step of supplying the tissue piece cryopreserving plate on which the tissue piece has been placed into a cooling medium tank.

The method of the present invention for cryopreserving the collected tissue by vitrification is effective when the method is used to cryopreserve an ovary tissue piece or a liver tissue piece buy vitrification.

The step of collecting the tissue piece is carried out as described above.

The step of immersing the tissue piece in a vitrification solution is carried out by immersing the tissue piece in a vitrification solution.

The following conventional vitrification solutions can be used. More specifically, vitrification solutions containing one kind or not less than two kinds of substances which permeate a cell membrane and protect a cell in freezing selected from among glycerol, propylene glycol, dimethyl sulfoxide(DMSO), ethylene glycol, butanediol, and the like and one kind or not less than two kinds of substances which do not permeate a cell membrane and protect a cell in freezing selected from among sucrose, trehalose, percoll, polyethylene glycol, polyvinyl pyrrolidone, bovine serum albumin, ficol, and the like.

It is preferable to perform treatment of immersing the collected tissue piece in an equilibrium liquid before the collected tissue piece is immersed in the vitrification solution. As the equilibrium liquid, a low-concentration solution of the substances which permeate a cell membrane and protect a cell in freezing is preferable. As the substances which permeate a cell membrane and protect a cell in freezing, those described above can be used.

After the step of immersing the collected tissue piece in the vitrification solution finishes, the tissue piece is taken out of the vitrification solution. Thereafter the step of placing the tissue piece on the tissue piece cryopreserving plate 4 is performed. The tissue piece is placed on the tissue piece cryopreserving plate 4 at a portion where the holes 42 are present

Thereafter the step of supplying the tissue piece cryopreserving plate on which the tissue piece has been placed into the cooling medium tank is performed. As the cooling medium, it is preferable to use a liquid nitrogen.

The appliance of the present invention for cryopreserving a collected tissue piece by vitrification includes the collected tissue cutting position-specifying tool and the tissue piece cryopreserving plate for cryopreserving a tissue piece cut from an organ tissue by the collected tissue cutting position-specifying tool. The collected tissue cutting position-specifying tool includes the tissue cutting portion-specifying member having the cutting portion-specifying open portion which is flat plate-shaped and has the predetermined width and the locking portion, for locking the advance of the cutter, which is provided on the lower surface of the tissue cutting portion-specifying member; and the open portion-pressing member which can be pressed against the upper surface of the tissue cutting portion-specifying member in such a way that the open portion-pressing member covers the cutting portion-specifying open portion. Therefore it is possible to collect the tissue piece having a desired size and thickness.

Further the tissue piece cryopreserving plate is the plate-shaped metal member which has a width equal to or a little larger than the predetermined width of the cutting portion-specifying open portion, is extended in a predetermined length, allows the tissue piece cut from the organ tissue by the collected tissue cutting position-specifying tool to be placed thereon, and has a lot of holes allowing a cooling medium to penetrate thereinto. Therefore it is possible to preferably freeze the tissue piece in a vitrified state by immersing the collected tissue piece in a vitrification solution, placing it on the tissue piece cryopreserving plate, and supplying the tissue piece cryopreserving plate on which the tissue piece has been placed into the cooling medium tank.

The tissue piece cryopreserving plate consists of a plate-shaped metal member which is extended in a predetermined length and has a tissue piece-placing portion having a lot of holes formed therein to allow a cooling medium to penetrate thereinto. Therefore it is possible to preferably freeze the tissue piece in a vitrified state by immersing the collected tissue piece in a vitrification solution, placing it on the tissue piece cryopreserving plate, and supplying the tissue piece cryopreserving plate on which the tissue piece has been placed into the cooling medium tank.

The method of the present invention for cryopreserving the collected tissue by vitrification is carried out by using the appliance for cryopreserving a collected tissue piece by vitrification.

The method of the present invention for cryopreserving a collected tissue piece by vitrification includes the step of notching the organ tissue along the inner edge of the cutting portion-specifying open portion with the cutting portion-specifying member being pressed against the organ tissue, and pressing the open portion-pressing member against the upper surface of the cutting portion-specifying member, and with the open portion-pressing member being pressed against the portion of the organ tissue surrounded with a notched line, moving the cutter along the lower surface of the cutting portion-specifying member so as to cut the tissue piece having the configuration corresponding to that of the cutting portion-specifying open portion from the organ tissue; the step of immersing the collected tissue piece in the vitrification solution; the step of taking out the tissue piece from the vitrification solution and placing the tissue piece on the tissue piece cryopreserving plate, and the step of supplying the tissue piece cryopreserving plate on which the tissue piece has been placed into the cooling medium tank. Therefore it is possible to preferably freeze tissue piece having a desired size and thickness in a vitrified state.

## Claims

1. An appliance for cryopreserving a collected tissue piece comprising:
a cutting position-specifying tool and a tissue piece cryopreserving plate for cryopreserving a tissue piece cut from an organ tissue by using said cutting position-specifying tool,
wherein said cutting position-specifying tool includes a tissue cutting portion-specifying member having a cutting portion-specifying open portion which is flat plate-shaped and has a predetermined width and a locking portion, for locking an advance of a cutter, which is provided on a lower surface of said tissue cutting portion-specifying member; and an open portion-pressing member which can be pressed against an upper surface of said tissue cutting portion-specifying member in such a way that said open portion-pressing member covers said cutting portion-specifying open portion; and
said tissue piece cryopreserving plate is a plate-shaped metal member which has a width equal to or a little larger than said predetermined width of said cutting portion-specifying open portion, is extended in a predetermined length, allows said tissue piece cut from said organ tissue by using said collected tissue cutting position-specifying tool to be paced thereon, and has holes allowing a cooling medium to penetrate thereinto.

2. An appliance according to claim 1, which is used to cryopreserve a tissue piece of an ovary by vitrification.

3. An appliance according to claim 1 or 2, wherein after an organ tissue is notched along an inner edge of said cutting portion-specifying open portion with said cutting portion-specifying member being pressed against said organ tissue, said open portion-pressing member is pressed against an upper surface of said cutting portion-specifying member in such a way that said open portion-pressing member covers said cutting portion-specifying open portion, and with said open portion-pressing member being pressed against a portion of said organ tissue surrounded with a notched line, a cutter is moved along a lower surface of said cutting portion-specifying member, whereby a tissue piece having a configuration corresponding to that of said cutting portion-specifying open portion can be cut from said organ tissue.

4. An appliance according to any one of claims 1 through 3, wherein said cutting portion-specifying open portion is rectangular; and said tissue piece cryopreserving plate has a width equal to or a little larger than that of said rectangular cutting portion-specifying open portion and has a length much longer than that of said rectangular cutting portion-specifying open portion.

5. An appliance according to any one of claims 1 through 3, wherein said cutting portion-specifying open portion is circular or elliptic; and said tissue piece cryopreserving plate has a width equal to or a little larger than a diameter or a minor axis of said open portion and has a length much longer than the diameter or a major axis of said open portion.

6. An appliance according to any one of claims 1 trough 5, wherein said locking portion for locking an advance of the cutter is disposed at one or other end of said tissue cutting portion-specifying member with respect to said cutting portion-specifying open portion.

7. An appliance according to any one of claims 1 through 6, wherein said locking portion for locking an advance of the cutter is disposed in said neighborhood of said cutting portion-specifying open portion.

8. An appliance according to any one of claims 1 through 7, wherein said open portion-pressing member is transparent flat plate-shaped.

9. An appliance according to any one of claims 1 through 8, wherein said tissue piece cryopreserving plate is an approximately rectangular thin plate and entirely has a lot of holes allowing a cooling medium to penetrate thereinto.

10. An appliance according to any one of claims 1 through 9, wherein said tissue piece cryopreserving plate is made of a metal selected from among copper, copper alloy, aluminum, aluminum alloy, gold, gold alloy, silver, and silver alloy.

11. An appliance according to any one of claims 1 through 10, wherein said tissue cutting portion-specifying member has an open portion-pressing member guide portion which is disposed at both sides of an upper surface thereof in a range from one longitudinal end thereof to other end thereof and slidably accommodates both sides of said open portion-pressing member.

12. An appliance according to any one of claims 1 through 11, wherein said tissue cutting portion-specifying member has a cutter guide portion disposed at least one widthwise end of a lower surface thereof in a range from one longitudinal end thereof toward other end thereof, thus allowing said cutter to slide thereon.

13. An appliance according to claim 12, wherein a distal side of said cutter guide portion is projected from one longitudinal end of said tissue cutting portion-specifying member.

14. An appliance, according to any one of claims 1 through 13, which is said appliance for cryopreserving a collected tissue piece is an appliance for vitrification cryopreserving a collected tissue piece.

15. A tissue piece cryopreserving plate consisting of a plate-shaped metal member which is extended in a predetermined length and has a tissue piece-placing portion having a lot of holes formed therein to allow a cooling medium to penetrate thereinto.

16. A tissue piece cryopreserving plate, according to claim 15, made of a metal selected from among copper, copper alloy, aluminum, aluminum alloy, gold, gold alloy, silver, and silver alloy.

17. A tissue piece cryopreserving plate, according to claim 15 or 16, which consists of an approximately rectangular thin plate and has a lot of holes allowing a cooling medium to penetrate thereinto.
